# EUROPÄISCHE PATENTANMELDUNG

(11) **EP 4 474 337 A1**
(43) Veröffentlichungstag der Anmeldung: **11.12.2024**
(21) Anmeldenummer: 23177507.3
(22) Anmeldetag: 06.06.2023
(51) Int. Cl.: B67B 7/92

(54) **VORRICHTUNG UND VERFAHREN ZUR ABGABE EINER FLÜSSIGEN AUSGANGSKOMPONENTE EINES KNOCHENZEMENTTEIGS**

(71) Anmelder: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(57) **Zusammenfassung**

Vorrichtung zur Abgabe einer flüssigen Ausgangskomponente eines Knochenzementteigs aus einer Glasampulle, umfassend
ein Gehäuse, eine in dem Gehäuse angeordnete Halterung zur Aufnahme einer Glasampulle und einen Auslass zur Abgabe der flüssigen Ausgangskomponente aus dem Gehäuse.

## Beschreibung

Die Erfindung betrifft eine Vorrichtung zur Abgabe einer flüssigen Ausgangskomponente eines Knochenzementteigs aus einer Glasampulle, umfassend
ein Gehäuse, eine in dem Gehäuse angeordnete Halterung zur Aufnahme einer Glasampulle und einen Auslass zur Abgabe der flüssigen Ausgangskomponente aus dem Gehäuse.

Die Erfindung betrifft weiterhin eine Mischvorrichtung umfassend eine derartige Vorrichtung und eine Mischeinheit sowie ein Verfahren zu Abgabe einer flüssigen Ausgangskomponente eines Knochenzementteigs mittels einer derartigen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind:
US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind beispielsweise in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In den vorgenannten Full-Prepacked-Systemen erfolgt die Vermischung einer Monomerflüssigkeit mit einem Knochenzementpulver durch mechanisches Vermischen, beispielsweise mittels eines Mischstabes.

Als besondere Herausforderung gestaltet sich bei den vorgenannten Systemen insbesondere die Bereitstellung der flüssigen Ausgangskomponente, insbesondere der Monomerflüssigkeit für einen PMMA-Knochenzementteig. Häufig findet die Lagerung dieser flüssigen Ausgangskomponente in Glasampullen statt, was insbesondere aufgrund der einfachen Sterilisierbarkeit solcher Glasampullen Vorteile bietet.

Zur Entnahme der flüssigen Ausgangskomponente aus einer handelsüblichen Glasampulle muss jedoch ein Ampullenkopf von einem Ampullenkörper der Glasampulle abgetrennt und entfernt werden. Zur Öffnung von Glasampullen in geschlossenen Vorrichtungen wurde eine Reihe von Patentschriften bekannt. Vorrichtungen zum Öffnen von Ampullen wurden beispielsweise in den Schriften DE19532015A1, WO9718031A1, und WO2010012114A1 offenbart.

Die Schrift EP2404864B1 offenbart eine Vorrichtung, bei der eine Ampulle in einem Ampullenhalter angeordnet ist und wobei im Bereich des Ampullenhalses der Ampullenhalter elastisch deformierbar ist. Im Bereich des Ampullenkopfs und des Ampullenkörpers ist der Ampullenhalter nicht deformierbar. Durch Knicken des Ampullenhalters über den elastisch deformierbaren Abschnitt des Ampullenhalters, der eine Scharnierfunktion hat, kann der Ampullenkopf gegen die nicht deformierbare Wandung des Ampullenhalters gedrückt werden. Dadurch kann der Ampullenkopf abbrechen und auf ein Sieb fallen. Die Monomerflüssigkeit kann in Folge aus der geöffneten Ampulle durch das Sieb fließen. Für die Funktion der dort beschriebenen Vorrichtung ist es vorteilhaft, dass diese mit einem mit Monomerflüssigkeit zu versorgenden Mischsystem mechanisch stabil verbindbar ist, weil eine erhebliche Zugbelastung beim Abknickvorgang an der Befestigung zum Mischsystem auftreten kann.

Es besteht im Markt der Wunsch nach weiteren Vorrichtungen, welche ein einfaches und sicheres Öffnen von Glasampullen zur Freisetzung flüssiger Ausgangskomponenten für Knochenzementteige erlaubt.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche ein einfaches, schnelles und anwendungssicheres, insbesondere verletzungsfreies, Öffnen einer oder mehrerer Glasampullen mit einer flüssigen Ausgangskomponente erlaubt. Insbesondere soll das Öffnen der Glasampulle oder der Glasampullen mit möglichst wenig Aufwand und unter Vermeidung zusätzlicher, separater Werkzeuge erfolgen. Weiterhin soll das Öffnen der Glasampulle mit möglichst wenigen Bauteilen ermöglicht sein. Weiterhin soll die flüssige Ausgangskomponente möglichst verlustfrei und schnell zum Anmischen eines Knochenzementteigs zur Verfügung stehen. Das Fördern der Monomerflüssigkeit aus der Vorrichtung zur Bereitstellung des Knochenzementteigs soll mit möglichst geringem Kraftaufwand durchführbar sein. Mittels der Vorrichtung soll es ermöglicht sein, mehrere, insbesondere zwei, Glasampullen gleichzeitig oder separat und zeitlich versetzt fluidleitend zu öffnen.

Die Vorrichtung soll mit möglichst wenigen Arbeitsschritten bedient werden können, um Fehlerquellen durch den Anwender zu minimieren.

Die Vorrichtung in Kombination mit einer Mischeinheit einsetzbar sein, um einen Knochenzementteig bereitstellen zu können.

Weiterhin soll eine flüssige Ausgangskomponente mittels der Vorrichtung steril und kontaminationsfrei bereitgestellt werden kann. Vorzugsweise soll ein Anwender der Vorrichtung nicht in Kontakt mit der zu öffnenden Glasampulle kommen.

Es ist eine weitere Aufgabe der Erfindung, eine Mischvorrichtung bereitzustellen, mittels welcher mindestens ein Teil der bereits beschriebenen Aufgaben zumindest zum Teil gelöst wird.

Es ist eine weitere Aufgabe der Erfindung, ein Verfahren zur Abgabe einer flüssigen Ausgangskomponente eines Knochenzementteigs bereitzustellen, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Vorrichtung zur Abgabe einer flüssigen Ausgangskomponente eines Knochenzementteigs aus einer Glasampulle, umfassend ein Gehäuse, eine in dem Gehäuse angeordnete Halterung zur Aufnahme einer Glasampulle einen Auslass zur Abgabe der flüssigen Ausgangskomponente aus dem Gehäuse, dadurch gekennzeichnet, dass
das Gehäuse einen verformbaren Bereich aufweist, welcher ausgebildet und eingerichtet ist, gegen die in der Halterung aufgenommene Glasampulle durch Druck verschiebbar zu sein und die Glasampulle fluidleitend zu öffnen, insbesondere so, dass die flüssige Ausgangskomponente in die Vorrichtung, insbesondere in das Gehäuse ausfließbar ist.

In einer Ausführungsform der Vorrichtung weist der verformbare Bereich des Gehäuses auf der der Haltung zugewandten Seite, also im Inneren des Gehäuses, ein im Wesentlichen formstabiles Druckstück auf, welches durch Druck gegen eine in der Halterung aufgenommene Glasampulle, insbesondere befüllt mit einer flüssigen Ausgangskomponente eines Knochenzementteigs, verschiebbar ist, insbesondere um diese durch diese Druckausübung fluidleitend zu öffnen. Diese Ausführungsform ist eine zweite Ausführungsform der Erfindung, welche vorzugsweise von der ersten Ausführungsform der Erfindung abhängt.

In einer Ausführungsform der Vorrichtung weist der verformbare Bereich einen Teilbereich, insbesondere einen umlaufenden Teilbereich, mit einer dünneren Wandstärke als das restliche Gehäuse auf. Diese Ausführungsform ist eine dritte Ausführungsform der Erfindung, welche vorzugsweise von der ersten oder zweiten Ausführungsform der Erfindung abhängt.

In einer Ausführungsform der Vorrichtung ist der verformbare Bereich zumindest abschnittsweise aus einem elastischen Material ausgeformt, insbesondere um ein im Wesentlichen vollständiges Rückstellen des verformbaren Bereichs nach dem fluidleitenden Öffnen einer Glasampulle in seine Ausgangsposition zu erlauben. Diese Ausführungsform ist eine vierte Ausführungsform der Erfindung, welche vorzugsweise von einer der vorhergehenden Ausführungsformen der Erfindung abhängt.

In einer Ausführungsform der Vorrichtung ist der verformbare Bereich zwischen der Halterung und dem Auslass angeordnet. Diese Ausführungsform ist eine fünfte Ausführungsform der Erfindung, welche vorzugsweise von einer der vorhergehenden Ausführungsformen der Erfindung abhängt.

In einer Ausführungsform der Vorrichtung ist die Halterung eingerichtet, eine Glasampulle derart in dem Gehäuse zu lagern, dass ein Ampullenkopf der Glasampulle an den verformbaren

Bereich des Gehäuses angrenzt. Diese Ausführungsform ist eine sechste Ausführungsform der Erfindung welche vorzugsweise von einer der vorgehenden Ausführungsformen der Erfindung abhängt.

In einer Ausführungsform der Vorrichtung ist zwischen der Halterung und dem Auslass ein Filterelement angeordnet, eingerichtet, um Bruchstücke einer in der Vorrichtung fluidleitend geöffneten Glasampulle in der Vorrichtung zurückzuhalten. Diese Ausführungsform ist eine siebte Ausführungsform der Erfindung, welche vorzugsweise von einer der vorhergehenden Ausführungsformen der Erfindung abhängt.

In einer Ausführungsform der Vorrichtung ist das Filterelement zwischen dem verformbaren Bereich und dem Auslass angeordnet. Diese Ausführungsform ist eine achte Ausführungsform der Erfindung, welche vorzugsweise von der siebten Ausführungsform der Erfindung abhängt.

In einer Ausführungsform der Vorrichtung ist zwischen der Halterung und dem Auslass ein Sammelbereich angeordnet, um eine flüssige Ausgangskomponente aus einer in der Vorrichtung fluidleitend geöffneten Glasampulle aufzunehmen. Diese Ausführungsform ist eine neunte Ausführungsform der Erfindung, welche vorzugsweise von einer der vorhergehenden Ausführungsformen der Erfindung abhängt.

In einer Ausführungsform der Vorrichtung, weist die Vorrichtung ein Fördermittel zum Fördern einer flüssigen Ausgangskomponente, insbesondere einer flüssigen Ausgangskomponente eines Knochenzementteigs, aus dem Sammelbereich durch den Auslass aus der Vorrichtung auf. Diese Ausführungsform ist eine zehnte Ausführungsform der Vorrichtung, welche vorzugsweise von der neunten Ausführungsform der Erfindung abhängt.

In einer Ausführungsform der Vorrichtung ist das Fördermittel ein Gummiballon. Diese Ausführungsform ist eine elfte Ausführungsform der Erfindung, welche vorzugsweise von der zehnten Ausführungsform der Erfindung abhängt.

In einer Ausführungsform der Vorrichtung ist das Fördermittel ein Kolben. Diese Ausführungsform ist eine zwölfte Ausführungsform der Erfindung, welche vorzugsweise von der zehnten Ausführungsform der Erfindung abhängt.

Eine dreizehnte Ausführungsform der Erfindung ist eine Mischvorrichtung zum Bereitstellen eines Knochenzementteigs aus zwei Ausgangskomponenten, umfassend eine Vorrichtung nach einer der ersten bis zwölften Ausführungsform der Erfindung und eine Mischeinheit, wobei die Mischeinheit ausgebildet und eingerichtet ist, aus den zwei Ausgangkomponenten den Knochenzementteig anzumischen, insbesondere anzumischen und somit bereitzustellen.

In einer Ausführungsform der Mischvorrichtung enthält die Vorrichtung eine Glasampulle mit einer flüssigen Ausgangskomponente als erste Ausgangskomponente des Knochenzementteigs und die Mischeinheit ein Knochenzementpulver als zweite Ausgangskomponente des Knochenzementteigs. Diese Ausführungsform ist eine vierzehnte Ausführungsform der Erfindung, welche vorzugsweise von der dreizehnten Ausführungsform der Erfindung abhängt.

Eine fünfzehnte Ausführungsform der Erfindung ist ein Verfahren zur Abgabe einer flüssigen Ausgangskomponente eines Knochenzementteigs aus einer Glasampulle mittels einer Vorrichtung nach einer der ersten bis zwölften Ausführungsform der Erfindung, umfassend die folgenden Schritte:
a. Bereitstellen einer Glasampulle enthaltend die flüssige Ausgangskomponente in der Halterung;
b. Fluidleitendes Öffnen der Glasampulle durch Druckausüben auf den verformbaren Bereich, so dass dieser gegen die Glasampulle gepresst wird;
c. Fördern der flüssigen Ausgangskomponente durch den Auslass aus der Vorrichtung.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Begriffe wie "proximal" und "distal" dienen lediglich der Bezeichnung der räumlich gegenüberliegenden Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zur Abgabe einer flüssigen Ausgangskomponente eines Knochenzementteigs aus einer Glasampulle, umfassend ein Gehäuse, eine in dem Gehäuse angeordnete Halterung zur Aufnahme einer Glasampulle, einen Auslass zur Abgabe der flüssigen Ausgangskomponente aus dem Gehäuse, dadurch gekennzeichnet, dass
das Gehäuse einen verformbaren Bereich aufweist, welcher ausgebildet und eingerichtet ist, gegen die in der Halterung aufgenommene Glasampulle durch Druck verschiebbar zu sein und die Glasampulle fluidleitend zu öffnen, insbesondere so, dass die flüssige Ausgangskomponente in die Vorrichtung, insbesondere in das Gehäuse ausfließbar ist.

Die Vorrichtung dient insbesondere einem fluidleitenden Öffnen einer oder mehreren in der Vorrichtung bereitgestellten Glasampulle beziehungsweise Glasampullen. Dazu weist das Gehäuse der Vorrichtung, insbesondere eine Gehäusewand des Gehäuses, einen verformbaren Bereich auf, welcher in ein Inneres des Gehäuses verschiebbar und somit gegen eine dort gelagerte Glasampulle pressbar ausgestaltet ist. Über den verformbaren Bereich kann somit von außerhalb der Vorrichtung, insbesondere durch einen Anwender der Vorrichtung, ein derart hoher Druck auf die in der Vorrichtung gelagerte Glasampulle ausgeübt werden, dass diese zumindest abschnittweise aufbricht und eine in der Glasampulle gelagerte flüssige Ausgangskomponente in das Gehäuse ausfließen kann. Dies erlaubt ein einfaches und schnelles fluidleitendes Öffnen einer Glasampulle ohne zusätzliche Hilfselemente. Zudem verringert sich durch ein derartiges Öffnen ohne direkten Kontakt mit der Glasampulle eine Verletzungsgefahr für den Anwender des herzustellenden Knochenzementteigs, insbesondere beim fluidleitenden Öffnen der Glasampulle. Vorzugsweise ist die Vorrichtung eingerichtet, die Glasampulle unter sterilen Bedingungen fluidleitend zu Öffnen und zum Anmischen des Knochenzementteigs bereitzustellen. Vorzugsweise ist das Gehäuse zur sterilen Lagerung einer Glasampulle eingerichtet. Vorzugsweise ist hierfür das Gehäuse im Wesentlichen von der Umgebung der Vorrichtung, vorzugsweise hermetisch, abgeschlossen.

Das Gehäuse der Vorrichtung dient der Aufnahme und Lagerung mindestens einer Glasampulle. Vorzugsweise sind in dem Gehäuse mehrere, vorzugsweise zwei, Glasampullen, vorzugsweise nebeneinander, insbesondere steril, transportsicher und vorzugsweise hermetisch von der Außenwelt, lagerbar.

Innerhalb des Gehäuses ist eine Halterung angeordnet, um mindestens eine, vorzugsweise zwei, Glasampullen aufzunehmen. Vorzugsweise umschließt die Halterung die mindestens eine Glasampulle zumindest abschnittsweise, so dass diese transportsicher in der Vorrichtung lagerbar ist. Die Halterung dient weiterhin einem Fixieren der mindestens einen Glasampulle, so dass diese mittels des verformbaren Bereichs durch Druckausübung fluidleitend zu öffnen ist.

Um eine flüssige Ausgangskomponente aus einer Glasampulle zum Anmischen eines Knochenzementteigs bereitzustellen, weist die Vorrichtung einen fluidleitenden Auslass, wie beispielsweise eine Durchführung, wie einen Schlauch oder ein Rohr, auf. Durch den Auslass kann die flüssige Ausgangskomponente nach dem fluidleitenden Öffnen der Glasampulle aus der Vorrichtung an eine gewünschte Stelle, insbesondere zum Anmischen eines Knochenzementteigs, bereitgestellt werden. Der Auslass kann an unterschiedlichen Stellen der Vorrichtung angeordnet sein, wobei es bevorzugt ist, diesen an einem distalen Ende, insbesondere an einem distalen Ende der Längsachse der Vorrichtung, anzuordnen.

Um ein fluidleitendes Öffnen einer in der Halterung gelagerten Glasampulle zu ermöglichen, kann der verformbare Bereich auf unterschiedliche Weise ausgestaltet sein, solange der verformbare Bereich in das Innere des Gehäuses derart einschiebbar ausgestaltet ist, dass über diesen von außerhalb der Vorrichtung Druck auf die Glasampulle ausgeübt werden kann, um die Glasampulle fluidleitend zu öffnen.

Beispielsweise kann der verformbare Bereich vollständig aus einem verformbaren Material ausgeformt sein.

In einer bevorzugten Ausführungsform können in der Vorrichtung mehr als eine Glasampulle in der Halterung aufgenommen werden und die Vorrichtung weist für jede dieser aufnehmbaren Glasampullen einen separaten verformbaren Bereich auf, so dass jeweils ein verformbarer Bereich genau eine Glasampulle fluidleitend öffnen kann. Dies erlaubt ein bedarfsgerechtes fluidleitendes Öffnen von Glasampullen. Beispielsweise weist die Vorrichtung in einer Ausführungsform eine Halterung für zwei Glasampullen und zwei separate verformbare Bereiche auf. Dabei sind die zwei verformbaren Bereiche so ausgestaltet, dass jeder der verformbaren Bereiche eine der zwei Glasampullen fluidleitend gezielt öffnen kann.

Eine Ausführungsform der Vorrichtung ist dadurch gekennzeichnet, dass der verformbare Bereich des Gehäuses zumindest auf der der Halterung zugewandten Seite, also auf der Innenseite des Gehäuses, ein im Wesentlichen formstabiles Druckstück aufweist, welches durch Druck, insbesondere gemeinsam mit dem verformbaren Bereich, gegen eine in der Halterung aufgenommene Glasampulle verschiebbar ist, um diese fluidleitend zu öffnen. Das Druckstück ist im Wesentlichen formstabil, also während der regulären Nutzung der Vorrichtung im Wesentlichen nicht durch den Anwender verformbar, was ein fluidleitendes Öffnen einer in der Halterung angeordneten Glasampulle durch Druckausübung auf den verformbaren Bereich erleichtert. Dazu ist das Druckstück so angeordnet, dass es spätestens unter Druckausübung auf den verformbaren Bereich in direkten Kontakt mit der zu öffnenden Glasampulle kommt. Weiterhin ist ein Anwender der Vorrichtung durch das Druckstück besser gegen mögliche Glasbruchstücke, welche beim fluidleitenden Öffnen einer Glasampulle entstehen können, geschützt. In einer Variante ist das Druckstück dabei zumindest an der der Halterung zugewandten Seite eines verformbaren Materials befestigt, beispielsweise geklebt oder form- und/oder kraftschlüssig in einer Einfassung befestigt, um einen verformbaren Bereich gemäß dieser Ausführungsform auszubilden. In einer weiteren Variante besteht der verformbare Bereich mittig aus dem Druckstück, welches umlaufend von einem verformbaren Material umgeben ist, um einen verformbaren Bereich gemäß dieser Ausführungsform auszubilden.

Der verformbare Bereich kann aus einem leichter zu verformenden Material als das restliche Gehäuse oder aus dem gleichen Material wie das restliche Gehäuse ausgeformt sein.

Eine Ausführungsform der Vorrichtung ist dadurch gekennzeichnet, dass der verformbare Bereich einen Teilbereich, insbesondere einen umlaufenden Teilbereich, mit einer dünneren Wandstärke als das restliche Gehäuse aufweist. Dies erlaubt die Fertigung des Gehäuses aus einem einheitlichen Material, wobei der verformbare Bereich leichter verformbar ist als der Rest des Gehäuses. In einer Variante weist das restliche Gehäuse eine derartige Wandstärke auf, welche, obwohl das Material an sich verformbar ist, im Wesentlichen unverformbar für einen Anwender der Vorrichtung ausgestaltet. In einer Variante weist der verformbare Bereich am umlaufenden Teilbereich, also an dem außenliegenden Teilbereich, welcher an das restliche Gehäuse angrenzt, eine dünnere Wandstärke auf als in seinem mittigen Teilbereich auf. Dabei kann der mittige Teilbereich als ein im Wesentlichen formstabiles Druckstück ausgestaltet sein. Vorzugsweise umfasst das Gehäuse samt verformbaren Bereich ein gummielastisches Material mit einer Shore Härte A von größer 60, insbesondere besteht das Gehäuse samt dem verformbaren Bereich aus einem derartigen Material. Beispiele für derartige Materialien sind biokompatible Elastomere, wie beispielsweise Silikon oder EPDM.

Eine Ausführungsform der Vorrichtung ist dadurch gekennzeichnet, dass der verformbare Bereich am umlaufenden Teilbereich eine faltenartige, insbesondere akkordeonartige, Struktur aufweist, welche ein Einschieben in Richtung der Halterung erlaubt. Dabei findet ein zumindest teilweises Entfalten dieser Falten statt.

Eine Ausführungsform der Vorrichtung ist dadurch gekennzeichnet, dass der verformbare Bereich zumindest abschnittsweise, vorzugsweise zumindest am umlaufenden Teilbereich, aus einem elastischen Material ausgeformt ist. Dies erlaubt ein selbstständiges Rückstellen des verformbaren Bereichs in seine Ausgangsstellung, oder zumindest in Richtung seiner Ausgangsstellung, nach einem fluidleitenden Öffnen einer in der Halterung gelagerten Glasampulle. So wird sichergestellt, dass der verformbare Bereich während der weiteren Verwendung der Vorrichtung, insbesondere beim Ausfließen oder Fördern der in der Glasampulle gelagerten flüssigen Ausgangskomponente, nicht störend im Inneren des Gehäuses verbleibt.

Der vorformbare Bereich kann an unterschiedlichen Stellen entlang der Längsachse des Gehäuses angeordnet sein.

Eine Ausführungsform der Vorrichtung ist dadurch gekennzeichnet, dass der verformbare Bereich räumlich zwischen der Halterung und dem Auslass angeordnet ist. So kann eine in der Halterung gelagerte Glasampulle in räumlicher Nähe des Auslasses fluidleitend geöffnet werden, ohne dass die flüssige Ausgangskomponente durch oder an der Halterung vorbei in Richtung des Auslasses fließen muss. Dies erleichtert ein im Wesentlichen vollständiges Fördern der flüssigen Ausgangskomponente aus der Vorrichtung.

Die erfindungsgemäße Vorrichtung ist insbesondere für Glasampullen ausgelegt, welche einen Ampullenkörper, einen Ampullenkopf und einen Ampullenhals, welcher den Ampullenkörper und den Ampullenkopf strukturell miteinander verbindet, aufweisen. Der Ampullenkörper stellt einen Großteil der Glasampulle dar und dient zur Aufnahme zumindest eines Großteils der flüssigen Ausgangskomponente in seinem Inneren. Der Ampullenhals weist einen im Gegensatz zum Ampullenkörper verringerten Außendurchmesser auf und dient als Sollbruchstelle zum Abbrechen des im Vergleich zum Ampullenkörpers kleinen Ampullenkopfes. Dem Fachmann sind derartige Glasampullen bestens bekannt.

Vorzugsweise ist die Halterung zur Aufnahme eines Ampullenhalses einer Glasampulle eingerichtet. Beispielsweise ist dazu die Halterung als eine Schulter, insbesondere ringartige Schulter, ausgeformt, wobei der Ampullenhals in einer Durchführung der Halterung angeordnet ist, der Ampullenkörper auf einer Seite der Schulter aufliegt und der Ampullenkopf auf der anderen Seite aus der Halterung herausragt.

Eine Ausführungsform der Vorrichtung ist dadurch gekennzeichnet, dass die Halterung eingerichtet ist, eine Glasampulle derart in dem Gehäuse zu lagern, dass ein Ampullenkopf der Glasampulle an den verformbaren Bereich des Gehäuses angrenzt. So kann der verformbare Bereich durch Druckausübung von außerhalb direkt auf den Ampullenkopf gepresst werden, welcher aufgrund der Sollbruchstelle der Glasampulle, dem Ampullenhals, erleichtert abbricht und so ein Ausfließen der in der Glasampulle gelagerten flüssigen Ausgangskomponente erlaubt. Diese Ausführungsform der Vorrichtung erleichtert einem Anwender das fluidleitende Öffnen einer derartigen Glasampulle.

Eine Ausführungsform der Vorrichtung ist dadurch gekennzeichnet, dass zwischen der Halterung und dem Auslass ein Filterelement angeordnet ist, eingerichtet, insbesondere eingerichtet und ausgestaltet, um Bruchstücke einer in der Vorrichtung fluidleitend geöffneten Glasampulle in der Vorrichtung zurückzuhalten. Das Filterelement verhindert somit eine Kontamination des anzumischenden Knochenzementteigs mit Bruchstücken einer Glasampulle. Um dies zu bewerkstelligen, wird eine Glasampulle vorzugsweise proximal des Filterelements angeordnet, während der Auslass distal des Filterelements angeordnet ist.

Das Filterelement kann beispielsweise ein Sieb, ein poröses Material oder eine Membran umfassen oder daraus bestehen. Vorzugsweise ist das Filterelement eine Porenscheibe, beispielsweise aus gesinterten Polypropylenpartikeln, aus gesinterten oder verpressten Polyethylenfasern, aus Cellulosefilz oder aus Pappe.

Eine Ausführungsform der Vorrichtung ist dadurch gekennzeichnet, dass das Filterelement zwischen dem verformbaren Bereich und dem Auslass angeordnet ist. Somit können Bruchstücke, welche durch fluidleitendes Öffnen einer Glasampulle entstehen können, wie beispielsweise ein abgebrochener Ampullenkopf, durch das Filterelement innerhalb der Vorrichtung zurückgehalten werden und gelangen nicht durch den Auslass aus der Vorrichtung.

Die Vorrichtung kann so ausgestaltet sein, dass eine flüssige Ausgangskomponente nach einem fluidleitenden Öffnen einer Glasampulle innerhalb der Vorrichtung direkt aus dieser herausfließen kann. Um eine verbesserte Kontrolle über den Zeitpunkt des Ausfließens der flüssigen Ausgangskomponente aus der Vorrichtung zu erlangen, kann es sinnvoll sein, die aus der Glasampulle ausfließende flüssige Ausgangskomponente zuerst innerhalb der Vorrichtung zu belassen und diese erst durch aktives Eingreifen aus dieser hinauszubefördern.

Um dies zu bewerkstelligen, ist eine Ausführungsform der Vorrichtung dadurch gekennzeichnet, dass zwischen der Halterung und dem Auslass ein Sammelbereich angeordnet ist, um eine flüssige Ausgangskomponente aus einer oder mehrerer in der Vorrichtung fluidleitend geöffneten Glasampulle beziehungsweise Glasampullen, vorzugsweise vollständig, aufzunehmen. Das Filterelement kann dabei proximal, also in Richtung der Glasampulle, distal oder auch mittig innerhalb des Sammelbereichs angeordnet sein. Vorzugsweise ist der Sammelbereich so ausgestaltet, dass neben der flüssigen Ausgangskomponente auch der oder die Ampullenköpfe der Glasampulle beziehungsweise Glasampullen aufgenommen werden können. Vorzugsweise ist der Sammelbereich dabei so dimensioniert, dass ein Ampullenkopf frei rotierbar im Sammelbereich lagerbar ist, so dass sich eventuell im Ampullenkopf befindliche flüssige Ausgangskomponente durch eine Drehung desselben ausfließen kann. Dies erleichtert ein im Wesentlichen vollständiges Fördern einer flüssigen Ausgangskomponente aus der Vorrichtung.

Ein Fördern einer flüssigen Ausgangskomponente aus der Vorrichtung, vorzugsweise aus dem Sammelbereich der Vorrichtung, durch den Auslass kann auf unterschiedlichen Weisen ausgelöst werden. Beispielsweise kann ein Fördern unter ausschließlicher Einwirkung der Schwerkraft ermöglicht sein. Dazu kann es notwendig sein, die Vorrichtung geeignet räumlich auszurichten, beispielsweise mit dem Auslass in Richtung des Bodens weisend.

Eine Ausführungsform der Vorrichtung ist dadurch gekennzeichnet, dass die Vorrichtung ein Fördermittel zum Fördern einer flüssigen Ausgangskomponente aus dem Sammelbereich durch den Auslass aus der Vorrichtung aufweist. Das Fördermittel dient dem aktiven Auslösen eines Fördervorgangs der flüssigen Ausgangskomponente durch einen Anwender der Vorrichtung.

Eine Ausführungsform der Vorrichtung ist dadurch gekennzeichnet, dass das Fördermittel ein Gummiballon ist. Ein Gummiballon es ein im weitesten Sinne blasenartiger Hohlkörper aus einem verformbaren, insbesondere elastischen, insbesondere reversibel elastischen, Material, mit einer fluidleitenden Öffnung. Um als Fördermittel zu fungieren, kann der Gummiballon über die fluidleitende Öffnung fluidleitend mit dem Inneren des Gehäuses verbunden sein oder verbunden werden, so dass durch ein Zusammendrücken des Gummiballons ein Förderdruck auf eine im Inneren des Gehäuses lagernde flüssige Ausgangskomponente ausübbar ist. Durch den Förderdruck kann die flüssige Ausgangskomponente aus dem Auslass der Vorrichtung gefördert werden. Zum Fördern der flüssigen Ausgangskomponente kann es erforderlich sein, den Gummiballon ein- oder mehrmalig zusammenzudrücken.

Vorzugsweise befindet sich der Gummiballon an einem dem Auslass entgegengesetztem Ende der Vorrichtung und ist dort fluidleitend mit dem Inneren des Gehäuses verbunden.

Eine Ausführungsform der Vorrichtung ist dadurch gekennzeichnet, dass das Fördermittel ein Kolben ist. Zum Fördern einer flüssigen Ausgangskomponente aus der Vorrichtung wirkt der Kolben mit einem Zylinder derart zusammen, dass ein Einschieben des Kolbens in den Zylinder einen Förderdruck auf eine im Inneren des Gehäuses lagernde flüssige Ausgangskomponente ausübt. Durch den Förderdruck kann die flüssige Ausgangskomponente aus dem Auslass der Vorrichtung gefördert werden.

In einer Variante dieser Ausführungsform fungiert das Gehäuse als Zylinder für den Kolben. In einer weiteren Variante dieser Ausführungsform ist der Zylinder fluidleitend mit dem Inneren des Gehäuses verbunden. Vorzugsweise ist der Zylinder an einem dem Auslass entgegengesetztem Ende der Vorrichtung angeordnet und dort mit dem Inneren des Gehäuses fluidleitend verbunden ist.

Ein weiterer Gegenstand der Erfindung betrifft eine Mischvorrichtung zum Bereitstellen eines Knochenzementteigs aus zwei Ausgangskomponenten, umfassend
eine Vorrichtung nach einer der vorhergehenden Ausführungsformen der Erfindung und eine Mischeinheit, wobei die Mischeinheit ausgebildet und eingerichtet ist, aus den zwei Ausgangskomponenten den Knochenzementteig anzumischen.

Die Mischeinheit dient einem Anmischen eines Knochenzementteigs aus einem Knochenzementpulver und der flüssigen Ausgangskomponente nach einem Fördern der flüssigen Ausgangskomponente in die Mischeinheit, insbesondere nach einem Fördern der flüssigen Ausgangskomponente in einen Innenraum der Mischeinheit.

Die Mischeinheit weist vorzugsweise eine hohlzylinderförmige Kartusche auf. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß kann die Kartusche aus unterschiedlichsten Materialien oder Materialkombinationen bestehen. Beispiele kann die Vorrichtung aus einem Polymer bestehen. Bevorzugt handelt es sich bei dem Polymer um ein transparentes Polymer, da der Anwender auf diese Weise eine ordnungsgemäße Funktion der Vorrichtung während einer Verwendung optisch kontrollieren kann.

Vorzugsweise ist in der Kartusche ein Austragskolben angeordnet, um den fertig angemischten Knochenzementteigs aus der Mischeinheit an eine gewünschte Stelle zu applizieren. Um den Knochenzementteig aus den beiden Ausgangskomponenten anzumischen, weist die Mischeinheit vorzugsweise ein Mischelement, wie beispielsweise eine Mischstange, auf. Alternativ kann ein Knochenzementteig in der Mischeinheit auch unter Schütteln derselben angemischt werden.

Die Vorrichtung und die Mischeinheit sind fluidleitend über den Auslass der Vorrichtung miteinander verbunden. In einer Variante sind die Vorrichtung und die Mischeinheit linear miteinander verbunden. In einer weiteren Variante sind die Vorrichtung und die Mischeinheit über ein fluidleitendes Leitungsmittel, wie beispielsweise einen Schlauch oder ein Rohr, fluidleitend miteinander verbunden. In einer Variante mündet der Auslass direkt in der Mischeinheit.

Die Vorrichtung und die Mischeinheit können fest, beispielsweise form- und/oder kraft- und/oder stoffschlüssig miteinander verbunden oder verbindbar sein. Ein einer Variante sind die Vorrichtung und die Mischeinheit einstückig ausgestaltet.

Eine Ausführungsform der Mischvorrichtung ist dadurch gekennzeichnet, dass die Vorrichtung eine Glasampulle mit einer flüssigen Ausgangskomponente als erste Ausgangskomponente des Knochenzementteigs und die Mischeinheit ein Knochenzementpulver als zweite Ausgangskomponente des Knochenzementteigs enthält. Dies erlaubt eine möglichst sterile Bereitstellung des Knochenzementteigs, da etwaige Umfüllschritte der Ausgangskomponenten durch einen Anwender der Mischvorrichtung entfallen und der Anwender bereits eine mit den Ausgangskomponenten steril vorbefüllte Mischvorrichtung erhält.

Ein weiterer Gegenstand der Erfindung betrifft ein Verfahren zur Abgabe einer flüssigen Ausgangskomponente eines Knochenzementteigs aus einer Glasampulle mittels einer Vorrichtung nach einer der vorhergehenden Ausführungsformen, vorzugsweise einer Vorrichtung als Bestandteil einer Mischvorrichtung nach einer der vorhergehenden Ausführungsformen der Erfindung, umfassend die folgenden Schritte:
a. Bereitstellen einer Glasampulle enthaltend die flüssige Ausgangskomponente in der Halterung;
b. Fluidleitendes Öffnen der Glasampulle durch Druckausüben auf den verformbaren Bereich, so dass dieser gegen die Glasampulle gepresst wird;
c. Fördern der flüssigen Ausgangskomponente durch den Auslass aus der Vorrichtung.

Das Bereitstellen der Glasampulle in Schritt a. kann durch einen Anwender der Vorrichtung direkt vor deren Verwendung geschehen oder der Anwender bekommt die mit der Glasampulle bestückte Vorrichtung bereits, beispielsweise durch den Hersteller der Vorrichtung, bereitgestellt.

Um die in der Halterung gelagerte Glasampulle fluidleitend zu öffnen, erfolgt in einem Schritt b. ein Pressen von außerhalb der Vorrichtung auf den verformbaren Bereich, welcher somit in oder weiter in den Innenraum des Gehäuses verschoben wird und letztendlich gegen die Glasampulle gepresst wird. Überschreitet dabei die Krafteinwirkung einen Grenzwert, kommt es zumindest zu einem teilweisen Bersten der Glasampulle, bevorzugt zu einem Abbrechen eines Ampullenkopfes der Glasampulle, wodurch diese fluidleitend geöffnet ist und die flüssige Ausgangskomponente in den Innenraum des Gehäuses ausfließen kann. Vorzugsweise ist der verformbare Bereich derart eingerichtet, dass dieser nach einem Beenden des Druckausübens wieder zumindest in Richtung, vorzugsweise vollständig, in seine Ausgangsstellung vor dem Schritt b. selbstständig zurückgeführt wird.

Vorzugsweise fließt die flüssige Ausgangskomponente nach dem fluidleitenden Öffnen der Glasampulle in einen Sammelbereich der Vorrichtung und verbleibt dort, bis der Anwender der Vorrichtung die flüssige Ausgangskomponente durch aktives Eingreifen aus der Vorrichtung fördert.

In einem Schritt c. erfolgt ein Fördern der flüssigen Ausgangskomponente durch den Auslass der Vorrichtung. In einer Variante des Schritts c. erfolgt das Fördern alleinig ausgelöst durch die Schwerkraft. In einer bevorzugten Variante des Schritts c. erfolgt das Fördern durch aktives Eingreifen des Anwenders der Vorrichtung. Beispielsweise betätigt der Anwender der Vorrichtung ein Fördermittel wie einen Kolben oder einen Gummiballon, um die flüssige Ausgangskomponente durch den Auslass der Vorrichtung zu fördern.

Vorzugsweise erfolgt das Fördern der flüssigen Ausgangskomponente in Schritt c. in eine mit der Vorrichtung fluidleitende verbundene Mischeinheit nach einer der vorhergehenden Ausführungsformen, in welcher die flüssige Ausgangskomponente mit einem Knochenzementpulver als eine zweite Ausgangskomponente des Knochenzementteigs in einem optionalen Schritt d. angemischt wird. Die Vorrichtung und die Mischeinheit bilden dabei gemeinsam eine Mischvorrichtung aus. Vorzugsweise kann der in der Mischeinheit bereitgestellte Knochenzementteig in einem optionalen Schritt e. direkt aus dieser an eine gewünschte Stelle ausgetragen werden. Vorzugsweise umfasst die Mischvorrichtung dazu einen Austragskolben, welcher axial innerhalb der Mischeinheit beweglich gelagert ist, um den bereitgestellten Knochenzementteig aus der Mischvorrichtung, insbesondere aus der Mischeinheit, auszutragen. Beispielsweise kann der Austragskolben dazu mittels einer Austragshilfe, wie beispielsweise einer Austragspistole, zum Austragen des Knochenzementteigs in der Mischeinheit verschoben werden.

Die Vorrichtung dient dem Bereitstellen einer flüssigen Ausgangskomponente für einen Knochenzementteig. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer flüssigen Ausgangskomponente, insbesondere einer Monomerflüssigkeit, als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Vorrichtung offenbarten Merkmale sind auch für die Mischvorrichtung sowie das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer beispielhaften Vorrichtung zur Abgabe einer flüssigen Ausgangskomponente eines Knochenzementteigs aus einer Glasampulle,
- Fig. 2: die Vorrichtung aus Figuren 1 mit fluidleitend geöffneter Glasampulle,
- Fig. 3: die Vorrichtung aus den Figuren 1 und 2 bei der Abgabe der flüssigen Ausgangskomponente,
- Fig. 4: einen schematischen Längsschnitt einer weiteren beispielhaften Vorrichtung zur Abgabe einer flüssigen Ausgangskomponente eines Knochenzementteigs aus einer Glasampulle umfassend ein Fördermittel in Form eines Gummiballons,
- Fig. 5: die Vorrichtung aus Figur 4 mit fluidleitend geöffneter Glasampulle,
- Fig. 6: die Vorrichtung aus den Figuren 4 und 5 bei der Abgabe der flüssigen Ausgangskomponente,
- Fig. 7: einen schematischen Längsschnitt einer weiteren beispielhaften Vorrichtung zur Abgabe einer flüssigen Ausgangskomponente eines Knochenzementteigs aus einer Glasampulle umfassend ein Fördermittel in Form eines Kolbens,
- Fig. 8: die Vorrichtung aus Figur 7 mit fluidleitend geöffneter Glasampulle,
- Fig. 9: die Vorrichtung aus den Figuren 7 und 8 bei der Abgabe der flüssigen Ausgangskomponente,
- Fig. 10: einen schematischen Längsschnitt einer beispielshaften Mischvorrichtung zum Bereitstellen eines Knochenzementteigs aus zwei Ausgangskomponenten, umfassend die Vorrichtung aus den Figuren 1 bis 3 und eine Mischeinheit,
- Fig. 11: einen schematischen Längsschnitt einer weiteren beispielhaften Mischvorrichtung zum Bereitstellen eines Knochenzementteigs aus zwei Ausgangskomponenten, umfassen eine Vorrichtung zur Abgabe einer flüssigen Ausgangskomponente eines Knochenzementteigs und eine Mischeinheit,
- Fig. 12: einen schematischen Längsschnitt einer weiteren beispielhaften Mischvorrichtung zum Bereitstellen eines Knochenzementteigs aus zwei Ausgangskomponenten, umfassen eine Vorrichtung zur Abgabe einer flüssigen Ausgangskomponente eines Knochenzementteigs und eine Mischeinheit, und
- Fig. 13: ein Flussdiagramm eines Verfahrens.

### Beschreibung der Figuren

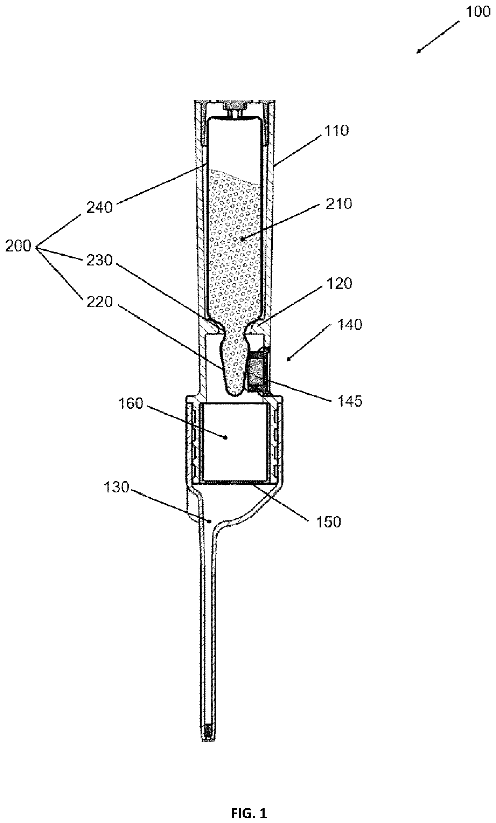

**Figur 1** zeigt einen schematischen Längsschnitt einer beispielhaften Ausführung einer Vorrichtung 100 zur Abgabe einer flüssigen Ausgangskomponente 210 eines Knochenzementteigs. Die Vorrichtung 100 umfasst ein zylinderartiges Gehäuse 110, in dem eine Glasampulle 200 beinhaltend die flüssige Ausgangskomponente 210 lagert. Die Glasampulle 200 umfasst einen Ampullenkörper 240, einen Ampullenkopf 220 und einen Ampullenhals 230 zwischen Ampullenkörper 240 und Ampullenkopf 220. Der Ampullenhals 230 ist in einer Halterung 120 der Vorrichtung 100 aufgenommen, um die Glasampulle 200 im Wesentlichen fest im Inneren des Gehäuses 110 zu fixieren. In der gezeigten Ausführungsform ist die Halterung 120 als eine Schulter ausgeformt, wobei der Ampullenkopf 220 auf einer Seite der Halterung 120 herausragt und der Ampullenkörper 240 auf der anderen Seite der Halterung 120 aufliegt.

Die Glasampulle 200 ist durch die Halterung 120 derart in dem Gehäuse 110 aufgenommen, dass der Ampullenkopf an einen verformbaren Bereich 140 des Gehäuses 110 angrenzt. Der verformbare Bereich 140 ist aus einem elastischen Material gefertigt und weist auf der der Halterung 120 zugewandten Seite ein im Wesentlichen formstabiles Druckstück 145 auf. Das Druckstück 145 weist somit in Richtung des Ampullenkopfes 220.

Um eine Abgabe der flüssigen Ausgangskomponente 210 aus der Vorrichtung 100 zu ermöglichen, weist diese einen Auslass 130 auf. In der gezeigten Ausführungsform der Vorrichtung 100 ist der Auslass 130 als ein Rohr ausgebildet. Räumlich zwischen dem Auslass 130 und der Halterung 120 ist im Inneren des Gehäuses 110 ein Filterelement 150 in Form einer Porenscheibe angeordnet, welches etwaige Bruchstücke der Glasampulle 200 nach einem Öffnen derselben in der Vorrichtung 100 zurückhalten kann. Räumlich zwischen dem Filterelement 150 und der Halterung 120 ist ein Sammelbereich 160 zur Aufnahme der flüssigen Ausgangskomponente 210 ausgebildet.

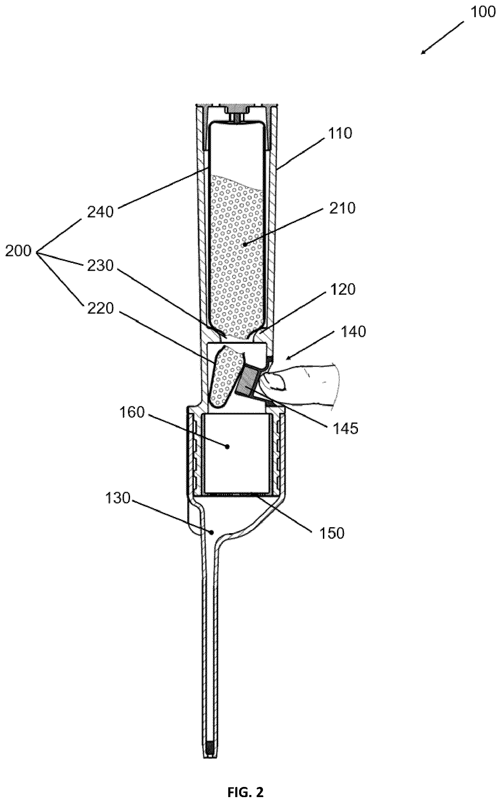

**Figur 2** zeigt die Vorrichtung 100 aus Figur 1 mit fluidleitend geöffneter Glasampulle 200. Zum Öffnen der Glasampulle 200 wurde der vorformbare Bereich 140 von außerhalb der Vorrichtung 100 in Richtung der Halterung 120 verschoben, so dass das Druckstück 145 gegen den Ampullenkopf 220 gepresst wurde. Durch diese Druckausübung wurde die strukturelle Integrität des Ampullenhalses 230, welcher als Sollbruchstelle der Glasampulle 200 fungiert, überschritten und die Glasampulle 200 durch ein Abbrechen des Ampullenkopfes 220 fluidleitend geöffnet.

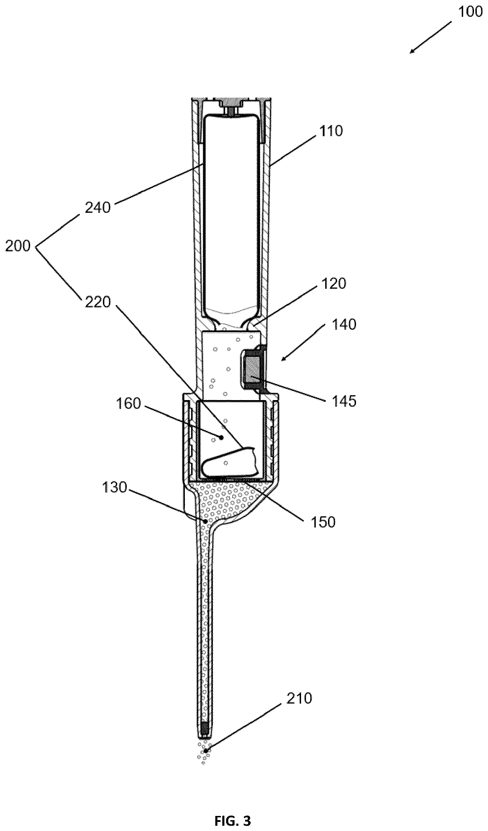

**Figur 3** zeigt die Vorrichtung 100 aus den Figuren 1 und 2 bei einer Abgabe der flüssigen Ausgangskomponente 210. Nach dem fluidleitenden Öffnen der Glasampulle 200 in Figur 2 ist der Ampullenkopf 220 gemäß der Schwerkraft in den Sammelbereich 160 gefallen und wurde dort vom Filterelement 150 zurückgehalten. Der Sammelbereich 160 ist so dimensioniert, dass der abgebrochene Ampullenkopf 220 in diesem frei rotierbar lagerbar ist, was ein möglichst vollständiges Ausfließen möglicherweise im Ampullenkopf 220 befindlicher, restlicher flüssiger Ausgangskomponente 210 erleichtert. Die flüssige Ausgangskomponente 210 ist aus der fluidleitend geöffneten Glasampulle 200 ausgelaufen, gemäß der Schwerkraft durch den Sammelbereich 160 und das Filterelement 150 geflossen und zumindest schon teilweise durch den Auslass 130 aus der Vorrichtung 100 ausgetreten.

Der in Figur 2 von außen auf den verformbaren Bereich 140 ausgeübt Druck wurde entfernt, so dass der verformbare Bereich 140 aufgrund seiner Materialeigenschaften im Wesentlichen wieder in seine ursprüngliche Ausgangsposition (vgl. Figur 1) zurückgekehrt ist.

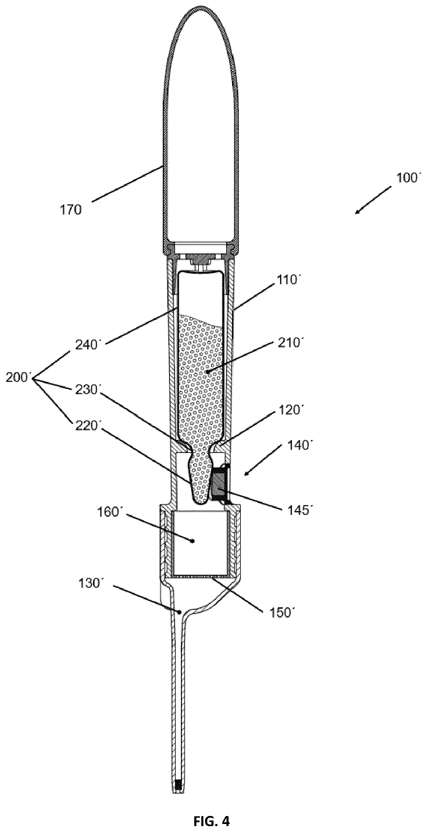

**Figur 4** zeigt einen schematischen Längsschnitt einer weiteren beispielhaften Ausführung einer Vorrichtung 100' zur Abgabe einer flüssigen Ausgangskomponente 210' eines Knochenzementteigs. Die Ausführungsform der Vorrichtung 100' stimmt weitgehend mit der vorstehend beschriebenen und in den Figuren 1 bis 3 dargestellten Ausführungsform überein, so dass zur Vermeidung von Wiederholungen auf die vorstehende Beschreibung verwiesen wird. Abwandlungen einer gegenüber der in den Figuren 1 bis 3 gezeigten Ausführungsform weisen dasselbe Bezugszeichen mit einem Apostroph auf.

Im Gegensatz zu der Ausführungsform der Figuren 1 bis 3, weist die Vorrichtung 100' ein Fördermittel 170 in Form eines Gummiballons auf. Das Fördermittel 170 ist an einem dem Auslass 130' entgegengesetztem axialen Ende des Gehäuses 110' angeordnet und mit dem Innenraum des Gehäuses 110' fluidleitend verbunden.

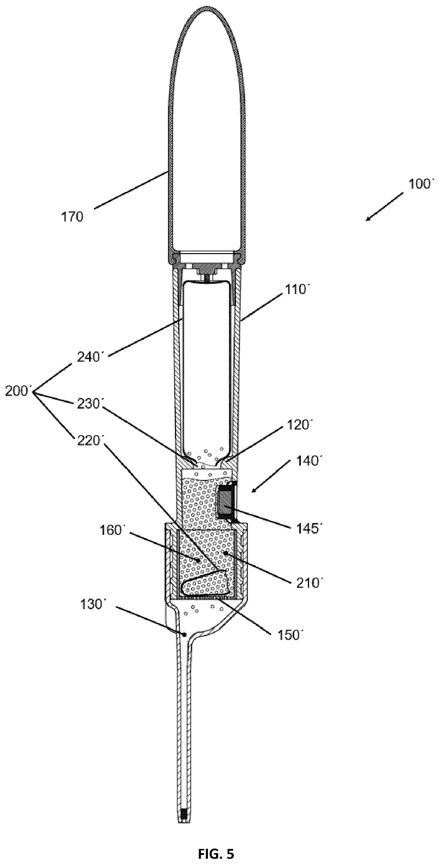

**Figur 5** zeigt die Vorrichtung 100' aus Figur 4 mit fluidleitend geöffneter Glasampulle 200'. Zum Öffnen der Glasampulle 200' wurde der vorformbare Bereich 140' von außerhalb der Vorrichtung 100' in Richtung der Halterung 120' verschoben, so dass das Druckstück 145' gegen den Ampullenkopf 220' gepresst wurde. Durch diese Druckausübung wurde die strukturelle Integrität des Ampullenhalses 230', welcher als Sollbruchstelle der Glasampulle 200' fungiert, überschritten und die Glasampulle 200' durch Abbrechen des Ampullenkopfes 220' fluidleitend geöffnet. Die flüssige Ausgangskomponente 210' ist aus der fluidleitend geöffneten Glasampulle 200' in den Sammelbereich 160' gemäß der Schwerkraft ausgeflossen, in welchem sie ohne Zutun eines Anwenders der Vorrichtung 100' vorerst verbleibt. Nach dem fluidleitenden Öffnen der Glasampulle 200' ist der Ampullenkopf 220' gemäß der Schwerkraft in den Sammelbereich 160' gefallen und wurde dort vom Filterelement 150' zurückgehalten. Der Sammelbereich 160' ist so dimensioniert, dass der abgebrochene Ampullenkopf 220' in diesem frei rotierbar lagerbar ist, was ein möglichst vollständiges Ausfließen möglicherweise im Ampullenkopf 220' befindlicher flüssiger Ausgangskomponente 210' erleichtert.

Der verformbare Bereich 140' ist nach dem fluidleitenden Öffnen der Glasampulle 200' aufgrund seiner Materialeigenschaften im Wesentlichen wieder in seine ursprüngliche Ausgangsposition (vgl. Figur 4) zurückgekehrt.

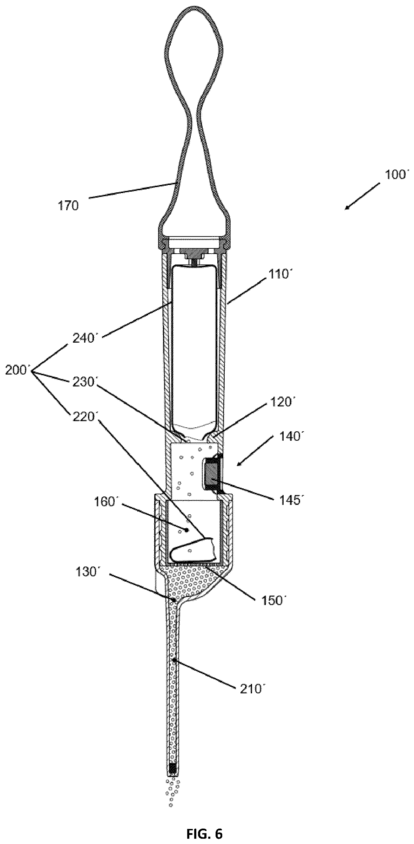

**Figur** 6 zeigt die Vorrichtung 100' aus den Figuren 4 und 5 bei einer Abgabe der flüssigen Ausgangskomponente 210' durch den Auslass 130'. Dazu wurde das Fördermittel 170 von außerhalb der Vorrichtung 100' zusammengedrückt, was einen Förderdruck auf die in dem Sammelbereich 160' gesammelte flüssige Ausgangskomponente 210' (vgl. Figur 5) ausgeübt und ein Vortreiben derselben durch den Auslass 130' ausgelöst hat. Zum vollständigen Austragen der flüssigen Ausgangskomponente 210' kann es erforderlich sein, das Fördermittel 170 mehrfach zu betätigen.

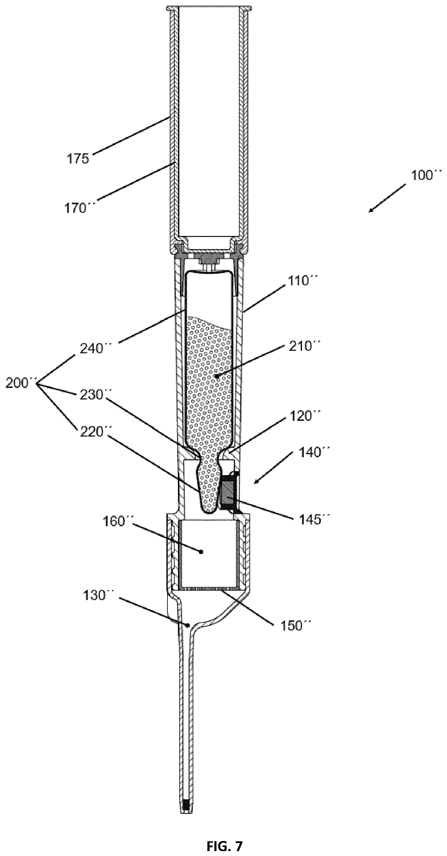

**Figur** 7 zeigt einen schematischen Längsschnitt einer weiteren beispielhaften Ausführung einer Vorrichtung 100" zur Abgabe einer flüssigen Ausgangskomponente 210" eines Knochenzementteigs. Die Ausführungsform der Vorrichtung 100" stimmt weitgehend mit den vorstehend beschriebenen und in den Figuren 1 bis 3 beziehungsweise 4 bis 6 dargestellten Ausführungsformen überein, so dass zur Vermeidung von Wiederholungen auf die vorstehenden Beschreibungen verwiesen wird. Abwandlungen einer gegenüber der in den Figuren 1 bis 3 beziehungsweise Figuren 4 bis 6 gezeigten Ausführungsform weisen dasselbe Bezugszeichen mit zwei Apostrophen auf.

Im Gegensatz zu der Ausführungsform der Figuren 1 bis 3, weist die Vorrichtung 100" ein Fördermittel 170" in Form eines Kolbens auf. Das Fördermittel 170 ist an einem dem Auslass 130" entgegengesetztem axialen Ende des Gehäuses 110" in einem Zylinder 175 des Gehäuses unter Ausbildung eines Kolben-Zylinder-Systems angeordnet, welcher mit dem die Glasampulle 220" beinhaltenden Innenraum des Gehäuses 110' fluidleitend verbunden ist.

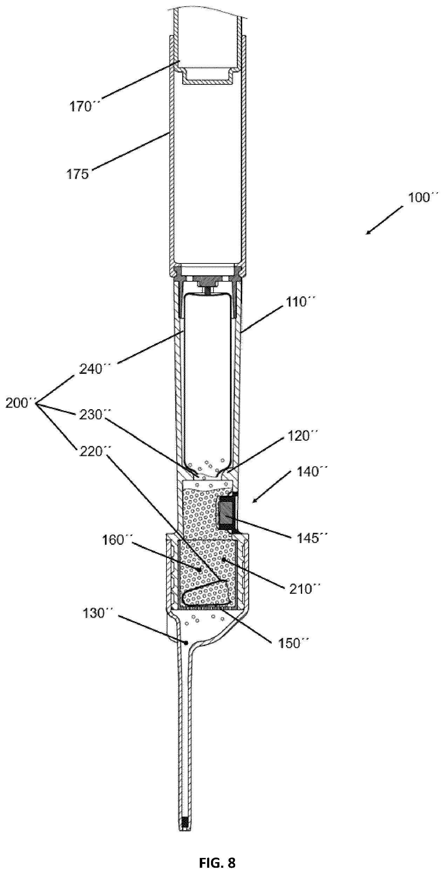

**Figur 8** zeigt die Vorrichtung 100" aus Figur 7 mit fluidleitend geöffneter Glasampulle 200". Zum Öffnen der Glasampulle 200' wurde der vorformbare Bereich 140" von außerhalb der Vorrichtung 100" in Richtung der Halterung 120" verschoben, so dass das Druckstück 145" gegen den Ampullenkopf 220" gepresst wurde. Durch diese Druckausübung wurde die strukturelle Integrität des Ampullenhalses 230", welcher als Sollbruchstelle der Glasampulle 200" fungiert, überschritten und die Glasampulle 200" durch eine Abbrechen des Ampullenkopfes 220" fluidleitend geöffnet. Die flüssige Ausgangskomponente 210" ist aus der fluidleitend geöffneten Glasampulle 200" in den Sammelbereich 160" gemäß der Schwerkraft ausgeflossen, in welchem sie ohne Zutun eines Anwenders der Vorrichtung 100" vorerst verbleibt. Nach dem fluidleitenden Öffnen der Glasampulle 200" ist der Ampullenkopf 220" gemäß der Schwerkraft in den Sammelbereich 160" gefallen und wurde dort vom Filterelement 150" zurückgehalten. Der Sammelbereich 160" ist so dimensioniert, dass der abgebrochene Ampullenkopf 220" in diesem frei rotierbar lagerbar ist, was ein möglichst vollständiges Ausfließen möglicherweise im Ampullenkopf 220" befindlicher, restlicher flüssiger Ausgangskomponente 210" erleichtert.

Der verformbare Bereich 140" ist nach dem fluidleitenden Öffnen der Glasampulle 200" aufgrund seiner Materialeigenschaften im Wesentlichen wieder in seine ursprüngliche Ausgangsposition (vgl. Figur 7) zurückgekehrt.

Um nachfolgend die flüssige Ausgangskomponente 220" aus dem Sammelbereich 160" durch den Auslass 130" zu fördern, wurde der Kolben 170" (lediglich abschnittsweise gezeigt) axial zum Teil aus dem Zylinder 175 herausgezogen.

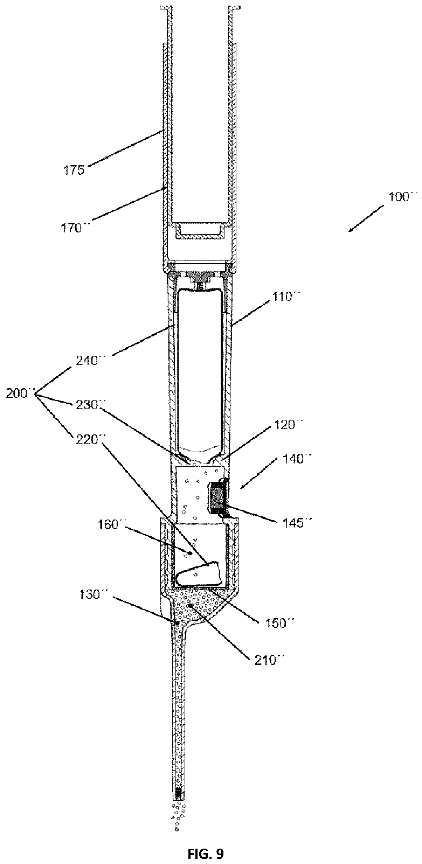

**Figur 9** zeigt die Vorrichtung 100" aus den Figuren 7 und 8 bei einer Abgabe der flüssigen Ausgangskomponente 210" durch den Auslass 130". Dazu wurde das Fördermittel 170 wieder in den Zylinder 175 eingeschoben, was einen Förderdruck auf die in dem Sammelbereich 160" gesammelte flüssige Ausgangskomponente 210" (vgl. Figur 8) ausgeübt und ein Vortreiben derselben durch den Auslass 130" ausgelöst hat. Zum vollständigen Austragen der flüssigen Ausgangskomponente 210" kann es erforderlich sein, das Fördermittel 170" mehrfach zu betätigen.

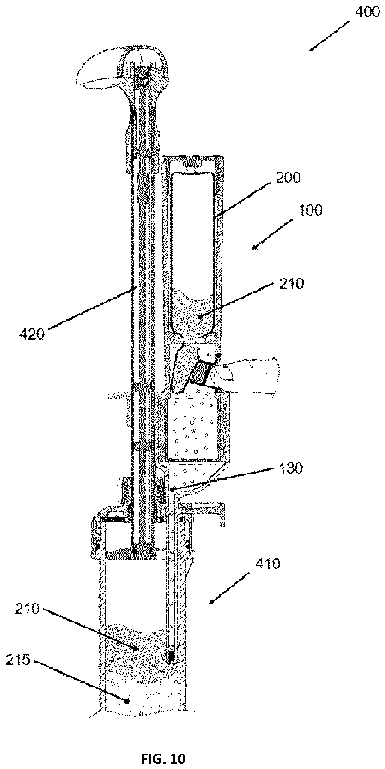

**Figur 10** zeigt einen schematischen Längsschnitt einer beispielshaften Mischvorrichtung 400 zum Bereitstellen eines Knochenzementteigs aus zwei Ausgangskomponenten, umfassend die Vorrichtung 100 aus den Figuren 1 bis 3 und eine Mischeinheit 410 (lediglich abschnittsweise gezeigt). Die Ausführungsform der Vorrichtung 100 stimmt weitgehend mit den vorstehend beschriebenen und in den Figuren 1 bis 3 dargestellten Ausführungsform überein, so dass zur Vermeidung von Wiederholungen auf die vorstehende Beschreibung verwiesen wird.

Die Vorrichtung 100 ist über den rohrartigen Auslass 130 reversibel fluidleitend mit der Mischeinheit 410 verbunden, wobei die flüssige Ausgangskomponente 210 nach einem fluidleitenden Öffnen der Glasampulle 200 bereits teilweise in die Mischeinheit 410 geflossen ist. Die Mischeinheit 410 enthält in einem Innenraum ein Knochenzementpulver 215 als zweite Ausgangskomponente des Knochenzementteigs. Um aus den Ausgangskomponenten 210, 215 den Knochenzementteig anzumischen, weist die Mischeinheit 410 ein axial im Inneren der Mischeinheit 410 reversibel bewegliches Mischelement 420 in Form eines Mischstabs auf. Das Mischelement 410 kann nach einem Entfernen der Vorrichtung 100 den Knochenzementteig durch wiederholtes axiales Verschieben im Innenraum der Mischeinheit 410 aus den Ausgangskomponenten 210, 215 bereitstellen.

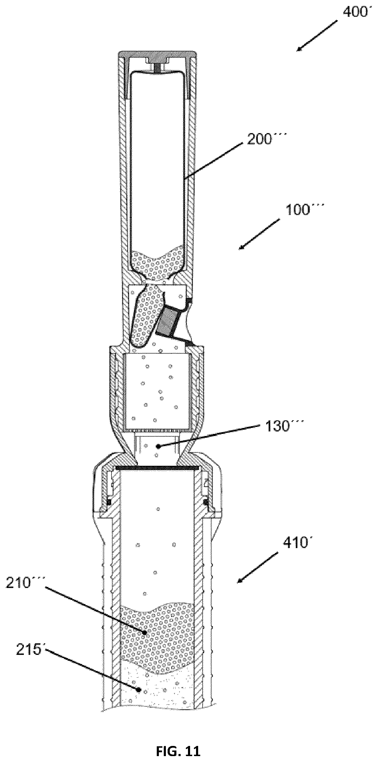

**Figur 11** zeigt einen schematischen Längsschnitt einer weiteren beispielhaften Mischvorrichtung 400' zum Bereitstellen eines Knochenzementteigs aus zwei Ausgangskomponenten, umfassend eine Vorrichtung 100‴ und eine Mischeinheit 410' (lediglich abschnittsweise gezeigt).

Die Ausführungsform der Vorrichtung 100‴ stimmt weitgehend mit den vorstehend beschriebenen und in den Figuren 1 bis 3 dargestellten Ausführungsform überein, so dass zur Vermeidung von Wiederholungen auf die vorstehende Beschreibung verwiesen wird. Abwandlungen einer gegenüber der in den Figuren 1 bis 3 gezeigten Ausführungsform weisen dasselbe Bezugszeichen mit drei Apostrophen auf.

Die Vorrichtung 100‴ ist in axialer Linie über den Auslass 130‴ fluidleitend mit dem Innenraum der Mischeinheit 410' verbunden, wobei die flüssige Ausgangskomponente 210‴ nach einem fluidleitenden Öffnen der Glasampulle 200‴ bereits teilweise in die Mischeinheit 410' geflossen ist. Die Mischeinheit 410' enthält in einem Innenraum ein Knochenzementpulver 215' als zweite Ausgangskomponente des Knochenzementteigs. Um es den Ausgangskomponenten den Knochenzementteig anzumischen, kann die Vorrichtung 100‴ reversibel von der Mischeinheit 410' abgetrennt werden und die beiden Ausgangskomponenten 210‴, 215' mit einem Mischelement (nicht gezeigt) vermischt werden.

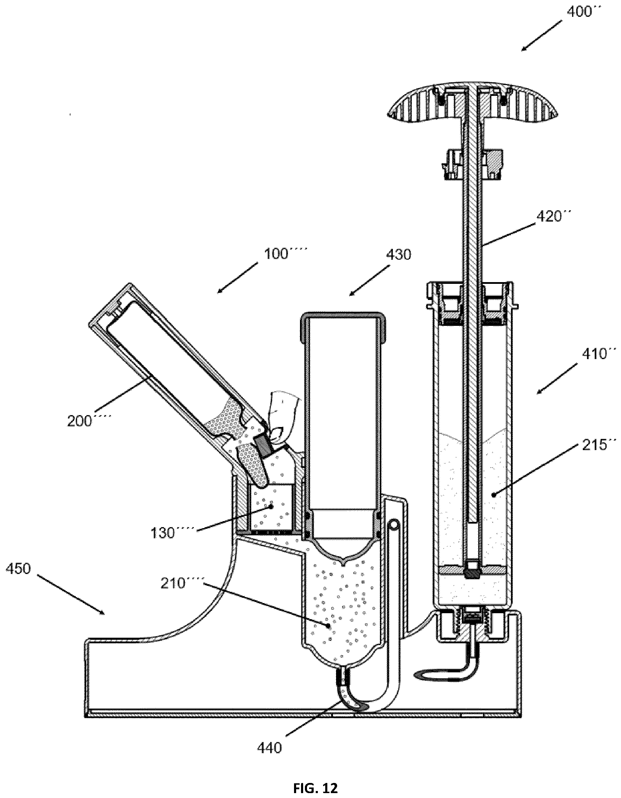

**Figur 12** zeigt einen schematischen Längsschnitt einer weiteren beispielhaften Mischvorrichtung 400" zum Bereitstellen eines Knochenzementteigs aus zwei Ausgangskomponenten, umfassend eine Vorrichtung 100ʺʺ und eine Mischeinheit 410".

Die Ausführungsform der Vorrichtung 100ʺʺ stimmt weitgehend mit den vorstehend beschriebenen und in den Figuren 1 bis 3 dargestellten Ausführungsform überein, so dass zur Vermeidung von Wiederholungen auf die vorstehende Beschreibung verwiesen wird. Abwandlungen einer gegenüber der in den Figuren 1 bis 3 gezeigten Ausführungsform weisen dasselbe Bezugszeichen mit vier Apostrophen auf.

Die Mischvorrichtung 410" umfasst weiterhin ein Standelement 450, über welches die Vorrichtung 100ʺʺ und die Mischeinheit 410" fluidleitend miteinander verbunden sind. Nach einem fluidleitenden Öffnen der Glasampulle 200ʺʺ fließt die flüssige Ausgangskomponente 210ʺʺ über den Auslass 130ʺʺ in ein Kolben-Zylinder-System 430 des Standelements 450 und wird dort gesammelt. Durch Betätigung des Kolben-Zylinder-Systems durch den Anwender der Mischvorrichtung 400" kann die flüssige Ausgangskomponente 210ʺʺ über einen Schlauch 440 in die Mischeinheit 410, insbesondere in ein dort lagerndes Knochenzementpulver 215", gefördert werden, um den Knochenzementteig durch Anmischen mittels eines Mischelements 420ʺʺ in Form einer Mischstange bereitzustellen. Zum Austragen des so bereitgestellten Knochenzementteigs kann die Mischeinheit 410" von dem Standelement 450 abgetrennt werden.

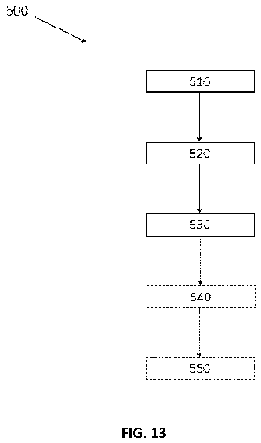

**Figur 13** zeigt ein Verfahren 500 zur Abgabe einer flüssigen Ausgangskomponente 210, 210', 210", 210‴, 210ʺʺ eines Knochenzementteigs aus einer Glasampulle 200, 200', 200", 200‴, 200ʺʺ mittels einer Vorrichtung 100, 100', 100", 100‴, 100ʺʺ gemäß den Figuren 1 bis 12 umfassend die Schritte 510 bis 530 sowie optional die Schritte 540 und 550.

In einem Schritt 510 wird mindestens eine Glasampulle 200, 200', 200", 200‴, 200ʺʺ in der Halterung 120, 120', 120" der Vorrichtung 100, 100', 100", 100‴, 100ʺʺ bereitgestellt. Dies kann von einem Anwender der Vorrichtung 100, 100', 100", 100‴, 100ʺʺ ausgeführt werden oder der Anwender erwirbt die Vorrichtung 100, 100', 100", 100‴, 100ʺʺ bereits mit einer oder mehreren vorbefüllten Glasampullen 200, 200', 200", 200‴, 200ʺʺ.

Um die in der Halterung 120, 120', 120" gelagerte Glasampulle 200, 200', 200", 200‴, 200ʺʺ fluidleitend zu öffnen, erfolgt in einem Schritt 520 ein Pressen von außerhalb der Vorrichtung 100, 100', 100", 100‴, 100ʺʺ auf den verformbaren Bereich 140, 140', 140", welcher somit in oder weiter in den Innenraum des Gehäuses 110, 110', 110" verschoben wird und letztendlich gegen die Glasampulle 200, 200', 200", 200‴, 200ʺʺ gepresst wird. Überschreitet dabei die Krafteinwirkung einen Grenzwert, kommt es zumindest zu einem teilweisen Bersten der Glasampulle 200, 200', 200", 200‴, 200ʺʺ, bevorzugt zu einem Abbrechen eines Ampullenkopfes 220, 220', 220" der Glasampulle 200, 200', 200", 200‴, 200ʺʺ, wodurch diese fluidleitend geöffnet wird und die flüssige Ausgangskomponente 210, 210', 210", 210'", 210ʺʺ in den Innenraum des Gehäuses 110, 110', 110" ausfließen kann. Vorzugsweise ist der verformbare Bereich 140, 140', 140"derart eingerichtet, dass dieser nach einem Beenden des Druckausübens wieder zumindest in Richtung, vorzugsweise vollständig, in seine Ausgangsstellung vor dem Schritt 520 selbstständig zurückgeführt wird.

Vorzugsweise fließt die flüssige Ausgangskomponente 210, 210', 210", 210‴, 210ʺʺ nach dem fluidleitenden Öffnen der Glasampulle 200, 200', 200", 200‴, 200ʺʺ in einen Sammelbereich 160, 160', 160" der Vorrichtung 100, 100', 100", 100‴, 100ʺʺ und verbleibt dort, bis der Anwender der Vorrichtung 100, 100', 100", 100‴, 100ʺʺ die flüssige Ausgangskomponente 210, 210', 210", 210‴, 210ʺʺ durch aktives Eingreifen aus der Vorrichtung 100, 100', 100", 100‴, 100ʺʺ fördert.

In einem Schritt 530 erfolgt ein Fördern der flüssigen Ausgangskomponente 210, 210', 210", 210‴, 210ʺʺ durch den Auslass 130, 130', 130", 130‴, 130ʺʺ der Vorrichtung 100, 100', 100", 100‴, 100ʺʺ. In einer Variante des Schritts 530 erfolgt das Fördern alleinig ausgelöst durch die Schwerkraft. In einer bevorzugten Variante des Schritts 530 erfolgt das Fördern durch aktives Eingreifen des Anwenders der Vorrichtung 100, 100', 100", 100‴, 100ʺʺ. Beispielsweise betätigt der Anwender der Vorrichtung 100, 100', 100", 100‴, 100ʺʺ ein Fördermittel 170, 170" wie einen Kolben oder einen Gummiballon, um die flüssige Ausgangskomponente 210, 210', 210", 210‴, 210ʺʺ durch den Auslass 130, 130', 130" der Vorrichtung 100, 100', 100", 100‴, 100ʺʺ zu fördern.

Vorzugsweise ist die Vorrichtung 100, 100', 100", 100‴, 100ʺʺ zusammen mit einer Mischeinheit 410', 410', 410" Bestandteil einer Mischvorrichtung 400, 400', 400", so dass das Fördern in Schritt 530 die flüssige Ausgangskomponente 210, 210', 210", 210‴, 210ʺʺ in die Mischeinheit 410', 410', 410" verbringt.

In einem optionalen Schritt 540 erfolgt ein Anmischen eines Knochenzementteigs in der Mischeinheit 410', 410', 410" der Mischvorrichtung 400', 400', 400" aus der flüssigen Ausgangskomponente 210, 210', 210", 210‴, 210ʺʺ und einem in der Mischeinheit 410, 410', 410" gelagerten Knochenzementpulver 215, 215', 215". Vorzugsweise weist dazu die Mischeinheit 410, 410" ein Mischelement 420, 420", vorzugsweise in Form einer Mischstange, auf.

In einem optionalen Schritt 550 wird der in der Mischvorrichtung 400, 400', 400" direkt aus dieser an eine gewünschte Stelle ausgetragen. Vorzugsweise umfasst die Mischvorrichtung 400, 400', 400" dazu einen Austragskolben, welcher axial innerhalb der Mischeinheit 410, 410', 410" beweglich gelagert ist, um den bereitgestellten Knochenzementteig aus der Mischvorrichtung 400, 400', 400", insbesondere aus der Mischeinheit 410, 410', 410", auszutragen.

**Bezugszeichen**
- 100, 100', 100", 100‴, 100ʺʺ: Vorrichtung
- 110, 110', 110": Gehäuse
- 120, 120', 120": Halterung
- 130, 130', 130" 130‴, 130ʺʺ: Auslass
- 140, 140', 140": verformbarer Bereich
- 145, 145', 145": Druckstück
- 150, 150', 150": Filterelement
- 160, 160', 160": Sammelbereich
- 170, 170": Fördermittel
- 175: Zylinder
- 200. 200', 200" 200‴, 200ʺʺ: Glasampulle
- 210, 210', 210" 210‴, 210ʺʺ: flüssige Ausgangskomponente
- 215, 215', 215": Knochenzementpulver
- 220, 220', 220": Ampullenkopf
- 230, 230', 230": Ampullenhals
- 240, 240', 240": Ampullenkörper
- 400, 400', 400": Mischvorrichtung
- 410, 410', 410": Mischeinheit
- 420, 420": Mischelement
- 430: Kolben-Zylinder-System
- 440: Schlauch
- 450: Standelement
- 500: Verfahren
- 510: Bereitstellen
- 520: fluidleitendes Öffnen
- 530: Fördern
- 540: Anmischen
- 550: Austragen

## Patentansprüche

1. Vorrichtung (100, 100', 100", 100‴, 100ʺʺ) zur Abgabe einer flüssigen Ausgangskomponente (210, 210', 210", 210‴, 210ʺʺ) eines Knochenzementteigs aus einer Glasampulle (200, 200', 200", 200‴, 200ʺʺ), umfassend
ein Gehäuse (110, 110', 110"), eine in dem Gehäuse (110, 110', 110") angeordnete Halterung (120, 120', 120") zur Aufnahme einer Glasampulle (200, 200', 200", 200‴, 200ʺʺ),
einen Auslass (130, 130', 130", 130‴, 130ʺʺ) zur Abgabe der flüssigen Ausgangskomponente (210, 210', 210", 210‴, 210ʺʺ) aus dem Gehäuse (110, 110', 110"),
**dadurch gekennzeichnet, dass**
das Gehäuse (110, 110', 110") einen verformbaren Bereich (140, 140', 140") aufweist, welcher ausgebildet und eingerichtet ist, gegen die in der Halterung (120, 120', 120") aufgenommene Glasampulle (200, 200', 200", 200‴, 200ʺʺ) durch Druck verschiebbar zu sein und die Glasampulle (200, 200', 200", 200‴, 200ʺʺ) fluidleitend zu öffnen.

2. Vorrichtung (100, 100', 100", 100‴, 100ʺʺ) nach Anspruch 1, **wobei** der verformbare Bereich (140, 140', 140") des Gehäuses (110, 110', 110") auf der der Halterung (120, 120', 120") zugewandten Seite ein im Wesentlichen formstabiles Druckstück (145, 145', 145") aufweist, welches durch Druck gegen die in der Halterung (120, 120', 120") aufgenommene Glasampulle (200, 200', 200", 200‴, 200ʺʺ) verschiebbar ist.

3. Vorrichtung (100, 100', 100", 100‴, 100ʺʺ) nach Anspruch 1 oder 2, **wobei** der verformbare Bereich (140, 140', 140") einen Teilbereich, insbesondere einen umlaufenden Teilbereich, mit einer dünneren Wandstärke als das restliche Gehäuse aufweist.

4. Vorrichtung (100, 100', 100", 100‴, 100ʺʺ) nach einem der vorhergehenden Ansprüche, **wobei** der verformbare Bereich (140, 140', 140") zumindest abschnittweise aus einem elastischen Material ausgeformt ist.

5. Vorrichtung (100, 100', 100", 100‴, 100ʺʺ) nach einem der vorhergehenden Ansprüche, **wobei** der verformbare Bereich (140, 140', 140") zwischen der Halterung (120, 120', 120") und dem Auslass (130, 130', 130", 130‴, 130ʺʺ) angeordnet ist.

6. Vorrichtung (100, 100', 100", 100‴, 100ʺʺ) nach einem der vorhergehenden Ansprüche, **wobei** die Halterung (120, 120', 120") eingerichtet ist, eine Glasampulle (200, 200', 200", 200‴, 200ʺʺ) derart in dem Gehäuse (110, 110', 110") zu lagern, dass ein Ampullenkopf (220, 220', 220") der Glasampulle (200, 200', 200", 200‴, 200ʺʺ) an den verformbaren Bereich (140, 140', 140") des Gehäuses (110, 110', 110") angrenzt.

7. Vorrichtung (100, 100', 100", 100‴, 100ʺʺ) nach einem der vorhergehenden Ansprüche, **wobei** zwischen der Halterung (120, 120', 120") und dem Auslass (130, 130', 130", 130‴, 130ʺʺ) ein Filterelement (150, 150', 150") angeordnet ist, eingerichtet, um Bruchstücke einer in der Vorrichtung (100, 100', 100", 100‴, 100ʺʺ) fluidleitend geöffneten Glasampulle (200, 200', 200", 200‴, 200ʺʺ) in der Vorrichtung (100, 100', 100", 100‴, 100ʺʺ) zurückzuhalten.

8. Vorrichtung (100, 100', 100", 100‴, 100ʺʺ) nach Anspruch 7, **wobei** das Filterelement (150, 150', 150") zwischen dem verformbaren Bereich (140, 140', 140") und dem Auslass (130, 130', 130", 130‴, 130ʺʺ) angeordnet ist.

9. Vorrichtung (100, 100', 100", 100‴, 100ʺʺ) nach einem der vorhergehenden Ansprüche, **wobei** zwischen der Halterung (120, 120', 120") und dem Auslass (130, 130', 130", 130‴, 130ʺʺ) ein Sammelbereich (160, 160', 160") angeordnet ist, um eine flüssige Ausgangskomponente (210, 210', 210", 210‴, 210ʺʺ) aus einer in der Vorrichtung (100, 100', 100", 100‴, 100ʺʺ) fluidleitend geöffneten Glasampulle (200, 200', 200", 200‴, 200ʺʺ) aufzunehmen.

10. Vorrichtung (100', 100") nach Anspruch 9, **aufweisend** ein Fördermittel (170, 170") zum Fördern einer flüssigen Ausgangskomponente (210, 210', 210", 210‴, 210ʺʺ) aus dem Sammelbereich (160', 160") durch den Auslass (130', 130") aus der Vorrichtung (100', 100").

11. Vorrichtung (100') nach Anspruch 10, **wobei** das Fördermittel (170') ein Gummiballon ist.

12. Vorrichtung (100") nach Anspruch 10, **wobei** das Fördermittel (170") ein Kolben ist.

13. Mischvorrichtung (400, 400', 400") zum Bereitstellen eines Knochenzementteigs aus zwei Ausgangskomponenten, umfassend
eine Vorrichtung (100, 100‴, 100ʺʺ) nach einem der Ansprüche 1 bis 12 und eine Mischeinheit (410, 410', 410"), wobei die Mischeinheit (410, 410', 410") ausgebildet und eingerichtet ist, aus den zwei Ausgangskomponenten den Knochenzementteig (300) anzumischen.

14. Mischvorrichtung (400, 400', 400") nach Anspruch 13, **wobei** die Vorrichtung (100, 100‴, 100ʺʺ) eine Glasampulle (200, 200‴, 200ʺʺ) mit einer flüssigen Ausgangskomponente (210, 210‴, 200ʺʺ) als erste Ausgangskomponente des Knochenzementteigs und die Mischeinheit (410, 410', 410") ein Knochenzementpulver (215, 215', 215") als zweite Ausgangskomponente des Knochenzementteigs (300) enthält.

15. Verfahren (500) zur Abgabe einer flüssigen Ausgangskomponente (210, 210', 210", 210‴, 210ʺʺ) eines Knochenzementteigs aus einer Glasampulle (200, 200', 200", 200‴, 200ʺʺ) mittels einer Vorrichtung (100, 100', 100", 100‴, 100ʺʺ) nach einem der Ansprüche 1 bis 12, umfassend die folgenden Schritte:
a. Bereitstellen (510) einer Glasampulle (200, 200', 200", 200‴, 200ʺʺ) enthaltend die flüssige Ausgangskomponente (210, 210', 210", 210‴, 210ʺʺ) in der Halterung (120, 120', 120");
b. Fluidleitendes Öffnen (520) der Glasampulle (200, 200', 200", 200‴, 200ʺʺ) durch Druckausüben auf den verformbaren Bereich (140, 140', 140"), so dass dieser gegen die Glasampulle (200, 200', 200", 200‴, 200ʺʺ) gepresst wird;
c. Fördern (530) der flüssigen Ausgangskomponente (210, 210', 210", 210‴, 210ʺʺ) durch den Auslass (130, 130', 130", 130‴, 130ʺʺ) aus der Vorrichtung (100, 100', 100", 100‴, 100ʺʺ).
